# EUROPÄISCHE PATENTANMELDUNG

(11) **EP 2 484 279 A1**
(43) Veröffentlichungstag der Anmeldung: **08.08.2012**
(21) Anmeldenummer: 12150657.0
(22) Anmeldetag: 10.01.2012
(51) Int. Cl.: A61B 5/026, G01F 1/708, G01F 1/712

(54) **Blutflusssensor**

(30) Priorität: 03.02.2011 US 201161438985 P
(71) Anmelder: BIOTRONIK SE & Co. KG, 12359 Berlin (DE)
(72) Erfinder: Skerl, Olaf, 18209 Bad Doberan (DE); Lippert, Michael, 91522 Ansbach (DE)
(74) Vertreter: Lindner-Vogt, Karin L.

(57) **Zusammenfassung**

Die Erfindung betrifft einen Blutflusssensor für die Messung einer Blutflussgeschwindigkeit in einem Blutgefäß eines lebenden Körpers. Erfindungsgemäß besitzt der Blutflusssensor
- einen Träger und wenigstens einen Sender (301,401) zum Aussenden von Wellen, deren Ausbreitung von zellulären Bestandteilen des Blutes gegenüber der Ausbreitung im Blutplasma abgelenkt werden, und
- zwei an dem Träger mit einem Abstand voneinander befestigten Empfangseinheiten (302,402) zum Empfangen von dem Sender ausgesandter Wellen,
die so angeordnet sind, dass die jeweils empfangenen Wellen einen voneinander verschiedenen, in Strömungsrichtung des Blutes zueinander versetzten Weg durch das Blut genommen haben,
Außerdem besitzt der Blutflusssensor
- eine Filtereinheit (303,403) zum Filtern eines Ausgangssignals einer jeweiligen Empfangseinheit derart, dass das gefilterte Ausgangssignal einen Rauschanteil des jeweiligen Ausgangssignals repräsentiert, und
- eine Kreuzkorrelationseinheit (500) zum Bestimmen eines Zeitversatzes zwischen dem gefilterten Ausgangssignal einer Empfangseinheit und dem gefilterten Ausgangssignal der jeweils anderen Empfangseinheit durch Korrelieren der gefilterten Ausgangssignale, wobei der Zeitversatz umgekehrt proportional zur jeweiligen Blutgeschwindigkeit ist.

## Beschreibung

Die Erfindung betrifft einen implantierbaren Blutflusssensor für die Messung einer Blutflussgeschwindigkeit in einem Blutgefäß eines lebenden Körpers. Die Blutflussgeschwindigkeit ist die Strömungsgeschwindigkeit des Blutes in einem Blutgefäß und wird im Folgenden auch kurz als Blutfluss oder Blutgeschwindigkeit bezeichnet.

Der Blutfluss in bestimmten Blutgefäßen ist ein wichtiger diagnostischer Parameter. Aus dem Blutfluss in der Aorta kann beispielsweise das effektive Schlagvolumen bestimmt werden und aus dem Zeitverlauf des Aortenflusses können Rückschlüsse auf den Grad einer Herzinsuffizienz gezogen werden, hämodynamische Auswirkungen von Arrhythmien oder Therapieparametern bestimmt oder Informationen über die Gefäßeigenschaften der Aorta gewonnen werden. Weiterhin ist beispielsweise der Blutfluss in den Nierenarterien ein wichtiger Parameter für die Diagnostik und Behandlung von Niereninsuffizienz.

Für die Messung des Blutflusses in Blutgefäßen ist eine Vielzahl an Methoden bekannt und teilweise in der Anwendung. Allgemein bekannt und breit angewendet sind akustische Verfahren, vorrangig nach dem Doppler-Effekt (Ultraschall-Doppler-Sonografie). Nachteilig für die Anwendung akustischer Doppler-Messung bei implantierbaren Systemen ist die Notwendigkeit, hierbei den Schallstrahl exakt und dauerhaft stabil auf das zu untersuchende Blutgefäß auszurichten. Geringe Abweichungen oder Schwankungen können zu nicht kompensierbaren Fehlmessungen führen. Außerdem muss der Schallstrahl beim Doppler-Verfahren in einem möglichst flachen Winkel zum Gefäß eingestrahlt werden, was die Positionierung weiter erschwert. Es sind auch Lösungen bekannt, bei denen der Schallstrahl senkrecht zum Gefäß verlaufen kann. In US 5,785,657 wird dazu beispielsweise die Autokorrelationsfunktion (AKF) des empfangenen Signals bestimmt. Aus den Minima der AKF soll die Zeit bestimmt werden, die die Streuteilchen für die Durchquerung des Messvolumens benötigen. Ist die Ausdehnung des Messvolumens exakt bekannt, lässt sich daraus deren Geschwindigkeit und damit die Durchflussgeschwindigkeit bestimmen. Nachteilig bei diesen Methoden ist wiederum, dass das sich ergebende Messvolumen exakt bekannt und dauerhaft stabil sein muss. Angewandt werden auch Verfahren nach der Ultraschall-Laufzeit-Methode, wobei zwei Schallwandler versetzt von außen am Blutgefäß angebracht werden. Nachteilig hierbei ist wiederum die notwendige exakte Ausrichtung der Schallwandler und die exakte Kenntnis und Langzeitstabilität des Abstandes zwischen ihnen. Auch die für dieses Verfahren erforderliche hochgenaue Messung der Laufzeit der Schallimpulse mit Genauigkeiten unter 1 ns (Nanosekunde) ist mit Langzeitimplantaten schwer zu realisieren.

Allgemein bekannt sind ebenfalls optische Verfahren nach dem Prinzip des Laser-Doppler-Anemometers, bei denen neben dem relativ großen technischen Aufwand hauptsächlich der hohe Energiebedarf nachteilig für die Verwendung bei Langzeitimplantaten ist. Alternative optische Verfahren, beispielsweise gemäß US 5,601,611, werten direkt die von den Blutteilchen zurückgestreuten Signale aus, um daraus mittels aufwändiger Rechenverfahren Informationen über die Blutströmungsgeschwindigkeit zu gewinnen. Auch hier ist der hohe technische Aufwand nachteilig für den Einsatz bei Langzeitimplantaten. Bekannt sind auch Verfahren, die das Photoplethysmogramm (PPG) zur Bestimmung von Surrogatparametern für Blutdruck und Blutfluss nutzen. Beispielsweise wird in US 2010/049060 ein PPG-Sensor beschrieben, der sich am Gehäuse eines implantierbaren medizinischen Gerätes (IMD), vorzugsweise in dessen Header, befindet. Es werden dort Methoden beschrieben, nach denen aus dem PPG Surrogatparameter für den Blutdruck und den Blutfluss bestimmt werden können. Nachteilig bei diesen Verfahren ist, dass ihnen Modellannahmen und Annahmen zu den Modellparametern zugrunde liegen, die nicht exakt bekannt sind und zeitlichen und individuellen Schwankungen unterliegen. Auch sekundäre Effekte, wie zum Beispiel Änderungen des Gefäßtonus, wirken sich stark auf die Bestimmung dieser Surrogatparameter aus.

Eine weitere große Klasse der Methoden zur Blutflussmessung sind die thermischen Methoden. Allgemein angewendet werden beispielsweise Thermodilutionsverfahren, aber auch die klassischen Verfahren der sogenannten Hitzdraht-Anemometrie sind weit verbreitet. Bekannt sind auch Korrelationsmethoden, bei denen dem Blutstrom ein thermisches Muster aufgeprägt wird und dann dessen Laufzeit über eine definierte Strecke gemessen wird. Nachteilig sind bei allen thermischen Methoden wiederum der hohe Energiebedarf und die Notwendigkeit eines deutlichen, lokalen Wärmeeintrags in das Blut.

Auch die Anwendung von sogenannten Härchen-Sensoren für die Blutflussmessung ist bekannt. Hierbei ragen kleine in MEMS-Technologie gefertigte Härchen oder Fähnchen in den Blutstrom, durch den sie verbogen oder ausgelenkt werden, siehe zum Beispiel US 2008/154141. Nachteilig hierbei ist das Hineinragen von Fremdkörpern in den Blutstrom, an denen sich Thromben bilden können. Auch können die Härchen oder Fähnchen verkleben oder verkapselt werden und dadurch ihre Funktion verlieren.

Der Erfindung liegt die Aufgabe zugrunde, einen alternativen Blutflusssensor zu schaffen.

Zur Lösung dieser Aufgabe wird ein Blutflusssensor für die Messung einer Blutflussgeschwindigkeit in einem Blutgefäß eines lebenden Körpers vorgeschlagen, der folgende Bestandteile hat:
- einen Träger und wenigstens einen Sender zum Aussenden von Wellen, deren Ausbreitung von zellulären Bestandteilen des Blutes gegenüber der Ausbreitung im Blutplasma abgelenkt werden, und
- zwei an dem Träger mit einem Abstand voneinander befestigten Empfangseinheiten zum Empfangen von dem Sender ausgesandter Wellen,
   wobei die Empfangseinheiten so angeordnet sind, dass die jeweils empfangenen Wellen im Einsatzfall einen voneinander verschiedenen, wenigstes in Strömungsrichtung des Blutes zueinander versetzten Weg durch das Blut genommen haben, sowie
- eine mit den Empfangseinheiten verbundenen Filtereinheit zum Filtern eines Ausgangssignals einer jeweiligen Empfangseinheit derart, dass das ein jeweiliges gefiltertes Ausgangssignal der Filtereinheit einen Rauschanteil eines jeweiligen Ausgangssignals einer jeweiligen Empfangseinheit repräsentiert, und
- eine mit der Filtereinheit verbundene Kreuzkorrelationseinheit zum Bestimmten eines Zeitversatzes zwischen dem gefilterten Ausgangssignals einer Empfangseinheit und dem gefilterten Ausgangssignal der jeweils anderen Empfangseinheit durch Korrelieren des gefilterten Ausgangssignals einer Empfangseinheit mit dem gefilterten Ausgangssignal der jeweils anderen Empfangseinheit, wobei der Zeitversatz umgekehrt proportional zur jeweiligen Blutgeschwindigkeit ist.

Der Träger und die daran befestigten Sender und Empfangseinheiten bilden zusammen vorzugsweise eine implantierbare Sensoranordnung.

Die Erfinder haben erkannt, dass ein aus US 3,762,221 grundsätzlich bekanntes Messprinzip sich aufgrund der Eigenschaften und der Zusammensetzung des Blutes auch für die Bestimmung der Blutgeschwindigkeit nutzen lässt.

Bekannt ist, dass im Blut Licht entsprechend der Dämpfungscharakteristik des Blutes in Abhängigkeit von der Wellenlänge unterschiedlich gedämpft wird. Die Dämpfungscharakteristik des Blutes ist darüber hinaus abhängig von dessen Sauerstoffbeladung. Bei der Photoplethysmografie (PPG) wird diese Eigenschaft genutzt, um die partielle Sauerstoffsättigung des Blutes zu bestimmen, indem man das Verhältnis der Lichtdämpfungen bei 2 bestimmten Wellenlängen misst. Aus einem Photoplethysmogramm kann bekanntermaßen auch die Blutdruckkurve abgeleitet werden, zum Beispiel durch den Einfluss der Blutdruckwelle auf die Dehnung der Blutgefäße.

Außerdem kann Blut vereinfacht als Emulsion betrachtet werden, in der die Blutteilchen (zum Beispiel Erythrozyten, Leukozyten) statistisch verteilt sind. Dadurch entsteht ein Rauschen, das dem Photoplethysmogramm-Signal überlagert ist. Für die klassischen Anwendungen wird dieses Rauschen durch Signalverarbeitungsmaßnahmen, wie zum Beispiel Filterung oder Mittelung mehrere Kurven, unterdrückt. Das jeweilige Rauschmuster entsteht durch die räumliche Verteilung der Blutteilchen über den Gefäßquerschnitt. Die Erfinder haben erkannt, dass die räumliche Verteilung der Blutteilchen in der Strömung über eine geringe Distanz weitgehend erhalten bleibt, und demnach auch das dadurch verursachte Rauschmuster. Die Erfinder haben weiter erkannt, dass indem ein Photoplethysmogramm an zwei eng benachbarten Orten gemessen und der Rauschanteil herausgelöst wird, mittels einer Kreuzkorrelation beider Rauschanteile die zeitliche Verschiebung des Rauschmusters bestimmt werden kann. Ist der Abstand der Messorte bekannt, kann mit der Zeitverschiebung des Rauschmusters die Strömungsgeschwindigkeit berechnet werden. Außerdem haben die Erfinder erkannt, dass die Blutflussgeschwindigkeitsmessung auch völlig unabhängig von einer Photoplethysmografie mit dem erfindungsgemäßen Blutfluss-sensor durchgeführt werden kann.

Der erfindungsgemäße Blutflusssensor beinhaltet eine implantierbare Sensoranordnung zur Bestimmung des Blutflusses in einem Blutgefäß. Die Sensoranordnung ist vorzugsweise an einer elastischen, um ein Blutgefäß zu legenden Manschette befestigt, die die Sensoren trägt. Eine solche Manschette wird im Folgenden auch als Sensormanschette bezeichnet. Ein implantierbares medizinisches Gerät (IMD) mit einer Elektronik zur Ansteuerung der Sensoren, Auswertung der Sensorsignale, Energieversorgung und Telemetrie kann ebenfalls an der Manschette angeordnet sein oder mit dieser über ein Kabel verbunden sein und ein abgesetztes Gehäuse aufweisen. An der elastischen Manschette sind mindestens zwei vorzugsweise identische oder zumindest ähnliche Sensoranordnungen in einem geeigneten definierten Abstand (zum Beispiel 1 cm) angebracht. Die Manschette selbst kann zusätzlich als Drucksensor dienen, indem sie ausgeführt ist, die Dehnung der Gefäßwand nach bekannten Methoden zu ermitteln. Durch die feste Anbringung der Sensoranordnungen an der Manschette ist der geometrische Abstand der Sensoranordnungen untereinander auch über lange Zeit hinreichend stabil.

Gemäß der unterschiedlichen Ausführungsvarianten kann eine Sensoranordnung jeweils die folgenden Bestandteile haben:
- Sender mit wenigstens 2 Lichtquellen geeigneter Wellenlänge (zum Beispiel Rot, 660 nm und Infrarot, 910 nm) und einer gegenüberliegenden optischen Empfangseinheit (zur Messung der Durchstrahlung des Blutstroms) oder
- Sender mit wenigstens 2 Lichtquellen geeigneter Wellenlänge (zum Beispiel Rot, 660 nm und Infrarot, 910 nm) und einer winkelversetzten, nicht gegenüberliegenden optischen Empfangseinheit (zur Messung der Reflexion/Streuung durch Blutteilchen im Blutstrom) oder
- Sender mit einer Lichtquelle geeigneter Wellenlänge (zum Beispiel Rot, 660 nm) und einer gegenüberliegenden optischen Empfangseinheit (zur Messung der Durchstrahlung) oder
- Sender mit einer Lichtquelle geeigneter Wellenlänge (zum Beispiel Rot, 660 nm) und einem winkelversetzten, nicht gegenüberliegenden optischen Empfangseinheit (zur Messung der Reflexion/Streuung).

Als Lichtquellen können geeignete Leuchtdioden (LEDs) direkt an der Manschette angebracht sein oder das Licht einer Lichtquelle wird über eine Lichtleitfaser (Beleuchtungsfaser) zugeführt. Bei Verwendung einer Lichtleitfaser ergibt sich die Möglichkeit, die Beleuchtungsfasem der einzelnen Sensoranordnungen an eine gemeinsame LED anzukoppeln und/oder mehrere Wellenlängen (mehrere LEDs) zeitlich getrennt in die Beleuchtungsfaser einzukoppeln. Die Ankopplung der Sensormanschette über Lichtleitfasern an das implantierbare medizinische gerät (IMD) hat den Vorteil, das keine elektrisch leitende Verbindung zwischen IMD und Sensormanschette besteht, allerdings entsteht durch die Verwendung der Lichtleitfasern ein größerer Verlust, der durch höheren Energieaufwand kompensiert werden muss.

Eine optische Empfangseinheit enthält jeweils ein geeignetes Element, das optische in elektrische Signale umwandelt, zum Beispiel eine Fotodiode. Diese Ausgangssignale der jeweiligen Empfangseinheit bzw. des jeweiligen Wandlerelementes einer jeweiligen Empfangseinheit werden im Folgenden auch als Empfangssignale bezeichnet. Auch die optische Empfangseinheit kann durch eine Lichtleitfaser (Empfangsfaser) mit der Sensoranordnung verbunden sein, wobei für jeden Empfangskanal eine separate Empfangsfaser verwendet wird.

Möglich ist auch die Verwendung einer einzigen Faser für die Beleuchtung und für den Empfang, wobei durch geeignete optische Mittel, zum Beispiel halbdurchlässige Spiegel, die eingekoppelten und die ausgekoppelten Strahlen getrennt werden. Die dabei entstehenden Verluste müssen allerdings wieder durch höheren Energieaufwand kompensiert werden.

Weiterhin kann die Strahlgeometrie durch geeignete optische Mittel, zum Beispiel Spaltblenden, günstig beeinflusst werden, um beispielsweise Störungen durch die benachbarte Sensoranordnung zu eliminieren oder den Messpfad örtlich begrenzt zu gestalten.

Aus den jeweiligen Empfangssignalen wird jeweils das Verhältnis der Dämpfungen (bzw. Reflexionsanteile) bei den unterschiedlichen Wellenlängen, oder die Dämpfung (Reflexionsanteil) bei einer Wellenlänge bestimmt oder es wird einfach nur das Empfangssignal selbst als Rohsignal für die weitere Signalverarbeitung verwendet. Aus diesem Rohsignal wird dann durch bekannte Mittel der Signalverarbeitung, beispielsweise durch Filterung mit einem Bandpass, der durch die Blutteilchen verursachte Rauschanteil herausgelöst, womit man für jeden Empfangskanal ein entsprechendes Rauschsignal erhält. Das bei Verwendung von mehreren Wellenlängen ebenfalls entstehende PPG-Signal wird für die erfindungsgemäße Lösung nicht benötigt, kann aber bei Bedarf für weitere diagnostische Verwendungen zur Verfügung gestellt werden. Aus den beiden Rauschsignalen wird mittels Kreuzkorrelation unter Anwendung bekannter Verfahren der Zeitversatz zwischen diesen beiden Rauschsignalen ermittelt. Da der geometrische Abstand der Sensoranordnungen bekannt ist, wird daraus die Strömungsgeschwindigkeit des Blutes sowohl im Betrag als auch in der Richtung bestimmt.

Die erfindungsgemäße Lösung ist nicht limitiert auf die Verwendung von Lichtwellen, sondern kann mit jeglichen Arten von Wellen realisiert werden, die durch die Blutteilchen in ihrer Ausbreitung gedämpft oder durch diese reflektiert bzw. gestreut werden, beispielsweise Ultraschall-Wellen oder elektromagnetische Wellen.

Die Werte für die Strömungsgeschwindigkeit können zur Unterdrückung von Störungen synchron zum Herzzyklus über eine bestimmte Anzahl von Herzzyklen gemittelt werden. Aus der Strömungsgeschwindigkeit können geeignete Parameter abgeleitet werden (zum Beispiel Mittelwerte, diastolischer Wert, systolischer Wert, Differenz zwischen diastolischen und systolischen Wert, Anstiege der Blutflusskurve zu bestimmten Zeiten im Herzzyklus, und so weiter).

Aus der Strömungsgeschwindigkeit oder den abgeleiteten Parametern können wieder Trends oder Trendparameter ermittelt werden, die mit Schwellwerten verglichen werden können um beispielsweise Alarme generieren zu können.

Es können Zusammenhänge zwischen Herzrate und Blutflussgeschwindigkeit ermittelt werden.

Neben der Möglichkeit, die beiden Sensoren in einer Manschette um ein Blutgefäß herum unterzubringen, kann man beide Sensoren auch auf einem Katheter oder einer implantierbaren Elektrode in der Blutbahn platzieren (zur Messung von Reflektion/Streuung). Das ermöglicht Blutflussmessung zum Beispiel in der Vena Cava, im Koronar-Sinus, oder zwischen rechten Atrium und rechten Ventrikel durch die Herzklappenebene (Mitralfluss).

Vorzugsweise ist der Blutflusssensor mit einer Steuer- und Auswerteeinheit verbunden, die dazu ausgebildet ist Werte für die Strömungsgeschwindigkeit des Blutes in Kombination mit weiteren Parametern auszuwerten. Besonders bevorzugt sind beispielsweise die folgenden Kombinationen:
In Kombination mit einem Elektrokardiogramm (EKG):
   - Zeitintervalle zwischen elektrischer Erregung des Herzens und resultierendem Blutfluss,
   - Triggerung der Blutflussmessung durch bestimmte Bedingungen, zum Beispiel bei einer Tachykardie.

In Kombination mit einem Beschleunigungssensor:
- Messung des Blutflusses bei Aktivität oder Ruhe, Unterschiede der entsprechenden Blutflussgeschwindigkeiten
- Messung im Stehen oder Liegen des Patienten, Unterschiede zwischen stehend und liegend
- Aufnahme der Herztöne und Zusammenhänge zwischen Herztönen und Blutfluss.
In Kombination mit einem Drucksensor: Zusammenhänge zw. Blutdruck und Blutfluss, um daraus zum Beispiel Aussagen über den Zustand der Blutgefäße gewinnen.

Implantierbare medizinische Geräte (IMD), die ausgebildet sind, ein Elektrokardiogramm aufzunehmen und/oder die einen Beschleunigungssensor aufweisen, sind grundsätzlich bekannt.

Mit Hilfe einer in den Blutflusssensor oder in ein mit dem Blutflusssensor verbundenes implantierbares medizinisches Gerät integrierten Telemetrieeinheit ist eine Datenübertragung an ein externes Gerät, beispielsweise ein Patientengerät möglich, die wiederum ein Telemonitoring und/oder die Anzeige und Auswertung erfasster Daten und Datenkombinationen in einem zentralen Service-Center und damit auch die Einbindung in ein Vorhersagemodell (Prädiktor) erlaubt. In einem zentralen Servicecenter können eine Vielzahl von Daten - auch solche die von Blutflusssensoren oder implantierbaren medizinischen Geräten verschiedener Patienten stammen - in Kombination miteinander ausgewertet werden, um so die Daten besser bewerten zu können und genauere Diagnosen und/oder Vorhersagen treffen zu können.

Mögliche Anwendungen oder Weiterbildungen des Blutflusssensors schließen die Folgenden ein:
- Überwachung der Perfusion von Organen, zum Beispiel der Niere
- Monitoring von Medikamentenwirkungen/-nebenwirkungen, Therapie-Optimierung
- als hämodynamischer Sensor, zum Beispiel für die CRT Therapieoptimierung oder als zusätzlicher Parameter für ein Herzfehler-Vorhersagemodell
- In Kombination mit Elektrokardiogramm (EKG): Bestimmung der elektromechanischen Kopplung im Herzen und daraus die Optimierung von Therapieparametern (zum Beispiel AV, VV-Delay)
- Bestimmung der hämodynamischen Auswirkungen einer Tachykardie oder Detektion einer Tachykardie, beispielsweise als zusätzliches Schockkriterium oder zur Minimierung abgegebener Schocks (zum Beispiel wenn trotz Tachykardie ein ausreichender Blutfluss detektiert wird)
- aus dem Mitralfluss können der E- und A-Welle aus der Echokardiographie äquivalente Parameter bestimmt werden, mit denen beispielsweise eine Optimierung der CRT-Therapie möglich ist
- Auswertung der Signalparameter des Rauschens selbst, zum Beispiel Abschätzung des Hämatokrit-Werts oder Parameter zur Größe/Größenverteilung der Blutteilchen.

Die Erfindung soll nun anhand eines Ausführungsbeispiels mit Bezug auf die Figuren näher erläutert werden. Diese zeigen in
- Fig. 1:: eine Prinzipskizze eines erfindungsgemäßen Blutflusssensors;
- Fig. 2:: zwei Prinzipskizzen (Seitenansicht und Querschnitt) zweier Sensoranordnungen auf einer elastischen Manschette für eine Blutflussmessung mittels Durchstrahlung;
- Fig. 3:: zwei Prinzipskizzen (Seitenansicht und Querschnitt) zweier Sensoranordnungen auf einer elastischen Manschette für eine Blutflussmessung mittels Erfassung gestreuter Strahlung;
- Fig. 4:: eine Prinzipskizze einer Sensoranordnung in Form eines in ein Blutgefäß einführbaren Katheters;
- Fig. 5:: eine Darstellung der unterschiedlichen Dämpfung von Licht verschiedener Wellenlänge bei verschiedener Blutsauerstoffsättigung.

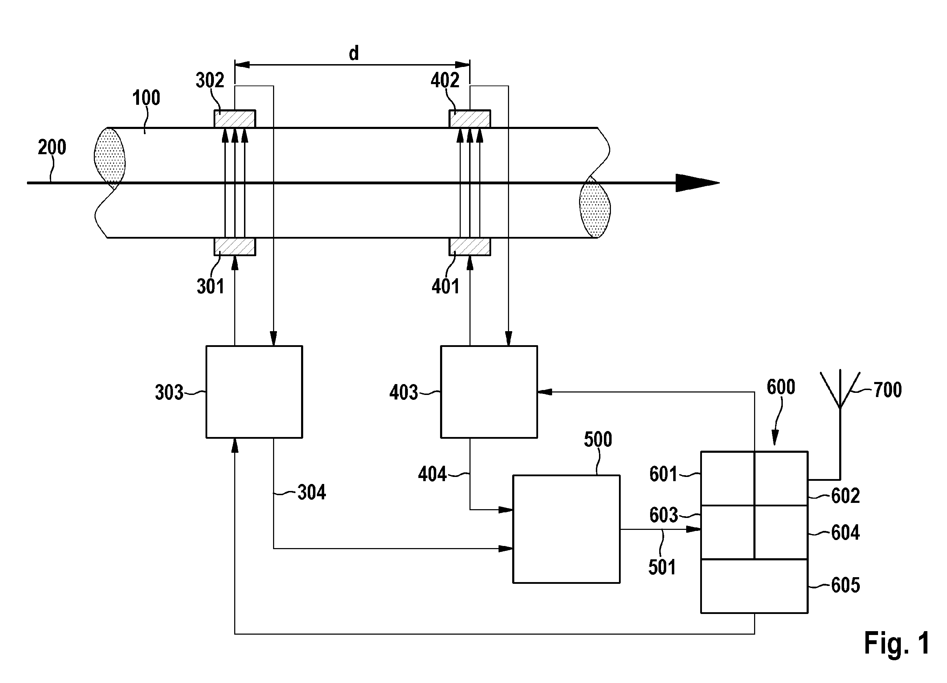

Fig. 1 zeigt eine beispielhafte Ausführungsvariante eines erfindungsgemäßen Blutfluss-Sensors. Dieser dient dazu, die Geschwindigkeit eines Blutstromes 200 in einem Blutgefäß 100 zu bestimmen. Hierzu sind zwei Sensoranordnungen mit jeweils einem Sender 301 bzw. 401 und einer Empfangseinheit 302 bzw. 402 vorgesehen. Die Empfangseinheit 302 der ersten Sensoranordnung ist dabei auf einer in dem Sender 301 der ersten Sensoranordnung gegenüberliegenden Seite des Blutgefäßes 100 angeordnet, so dass von dem Sender 301 ausgestrahltes rotes oder infrarotes Licht den Blutstrom 200 quer durchstrahlt und anschließend auf die Empfangseinheit 302 trifft. Die zweite Sensoranordnung mit dem Sender 401 und der Empfangseinheit 402 ist ähnlich aufgebaut wie die erste Sensoranordnung mit dem Sender 301 und der Empfangseinheit 302 und in einem Abstand d in Längsrichtung des Blutgefäßes 100 gegenüber der ersten Sensoranordnung versetzt. Ein geeigneter Abstand d hat die Größenordnung von einigen mm bis hin zu wenigen cm.

Ein jeweiliger Sender 301 bzw. 401 enthält eine Lichtquelle, beispielsweise eine Leuchtdiode (LED), zum Aussenden von rotem oder infrarotem Licht. Die jeweilige Lichtquelle muss dabei nicht immer direkt an das Blutgefäß 100 angrenzend angeordnet sein. Sie kann auch über eine in Fig. 1 nicht dargestellte Lichtleitfaser Licht in das Blutgefäß 100 einkoppeln.

Eine jeweilige Empfangseinheit 302 bzw. 402 besitzt ein Wandlerelement, welches zum Empfangen von einem jeweiligen Sender ausgesandten Lichts und dessen Umwandlung in ein elektrisches Ausgangsignal, welches auch als Empfangssignal bezeichnet wird, ausgebildet ist. Ein geeignetes Wandlerelement ist beispielsweise eine Fotodiode. Auch ein solches Wandlerelement braucht nicht unbedingt unmittelbar an das Blutgefäß 100 angrenzend angeordnet sein, sondern kann vielmehr ebenfalls über eine Lichtleitfaser an das Blutgefäß 100 optisch angekoppelt sein, um so Licht aus dem Blutgefäß 100 auszukoppeln.

Jede der Sensoranordnungen 301, 302 bzw. 401, 402 ist jeweils mit einer Sensorsteuer-und Filtereinheit 303 bzw. 403 verbunden. Die jeweilige Sensorsteuer- und Filtereinheit ist zum Ansteuern des jeweiligen Sensors 301 bzw. 401 sowie zum Aufnehmen und Auswerten des jeweiligen Empfangssignals der Empfangseinheit 302 bzw. 402 ausgebildet. Die Aufbereitung und Filterung des jeweiligen Empfangssignals durch die jeweilige Sensorsteuer- und Filtereinheit schließt eine Filterung, vorzugsweise eine Bandpassfilterung, des jeweiligen Empfangssignals ein, um auf diese Weise ein Sensorausgangssignal 304 bzw. 404 zu generieren, dessen Signalform wesentlich vom Rauschanteil des jeweiligen Empfangssignals bestimmt ist.

Die beiden auf diese Weise zu gewinnenden Sensorausgangssignale 304 und 404 werden einer Kreuzkorrelationseinheit 500 zugeführt, die die beiden von den zwei verschiedenen Sensoranordnungen stammenden Sensorausgangssignale 304 und 404 einer Kreuzkorrelation unterzieht, deren Ergebnis ein Zeitversatz zwischen den beiden Sensorausgangssignalen ist, um den zeitlich versetzt ähnliche Signalmerkmale dieser Sensorausgangssignale zeitlich zueinander versetzt im jeweils anderen Sensorausgangssignal wieder erscheinen. Dieser Zeitversatz entspricht der Zeit, die der Blutstrom 200 braucht, um die Distanz d, die die beiden Sensoranordnungen 301, 302 und 401, 402 in Strömungsrichtung des Blutstromes 200 zueinander versetzt sind, zurückzulegen. Die Kreuzkorrelationseinheit 500 generiert somit ein Zeitversatzsignal 501, das quasi einer durch den Blutstrom bewirkten Signallaufzeit über die Distanz d entspricht. Das Zeitversatzsignal 501 wird einer Steuer- und Auswerteeinheit 600 zugeführt, die dazu ausgebildet ist, aus der Signallaufzeit bei der bekannten Distanz d den Blutfluss, also die Fließgeschwindigkeit des Blutes, über die Distanz d zu berechnen. Die Steuer- und Auswerteeinheit 600 steuert außerdem die beiden Sensorsteuer- und Filtereinheiten 303 und 403 an. Sie enthält hierzu einen Mikrocontroller 601 sowie eine Telemetrieeinheit 602, mit der erfasste Werte über eine Telemetrieantenne 700 an ein externes Gerät übertragen werden können oder über die von einem externen Gerät Steuerbefehle empfangen werden können. Erfasste Werte oder Steuerbefehle können hierzu in einem Speicher 603 der Steuer- und Auswerteeinheit 600 zwischengespeichert werden. Eine Ablaufsteuerung 604 enthält schließlich die Steuerbefehle, die den Betrieb der Steuer- und Auswerteeinheit 600 steuern. Da die Steuer- und Auswerteeinheit als elektronische Steuerung ausgebildet ist, weist sie auch eine Energieversorgung 605, beispielsweise in Form einer Batterie, auf.

Die Steuer- und Auswerteeinheit 600 kann, wie bereits angedeutet, auch mit weiteren Sensoren oder anderen Steuereinheiten verbunden sein, so dass die hier konkret beschriebenen, den Blutfluss charakterisierenden Signale des Blutflusssensors mit weiteren Signalen, insbesondere Signalen eines implantierbaren Herzstimulators (beispielsweise eines Herzschrittmachers oder eines Kardioverters/Defibrillators) oder aber auch Signalen eines Drucksensors kombiniert und in Kombination ausgewertet werden können.

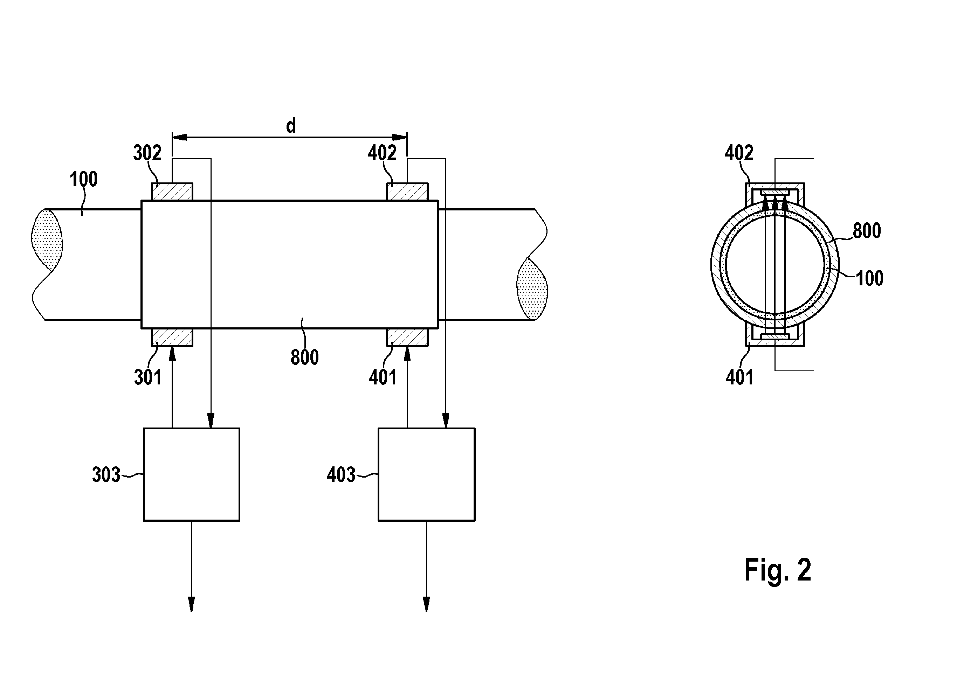

Fig. 2 zeigt schematisch, wie die beiden Sensoranordnungen mit ihrem jeweiligen Sender 301 bzw. 401 und ihrer jeweiligen Empfangseinheit 302 bzw. 402 an einer elastischen Manschette 800 befestigt sein können, welche um ein Blutgefäß 100 herumgelegt werden kann. Die beiden Sensoren sind auf dieser elastischen Manschette 800 in einem Abstand d voneinander angeordnet und zwar so, dass jede der beiden Sensoranordnungen das Blutgefäß 100 quer durchleuchtet (siehe den Querschnitt rechts in Fig. 2). Nicht dargestellt ist, dass die elastische Manschette 800 als Drucksensor ausgebildet ist, in dem Mittel vorgesehen sind, die dazu geeignet sind, aus der elastischen Deformation der Manschette 800 ein den pulsierenden Blutstrom repräsentierendes Drucksignal zu generieren. Ein solches Drucksignal eines Drucksensors kann zusammen mit dem Blutflusssignal ausgewertet werden.

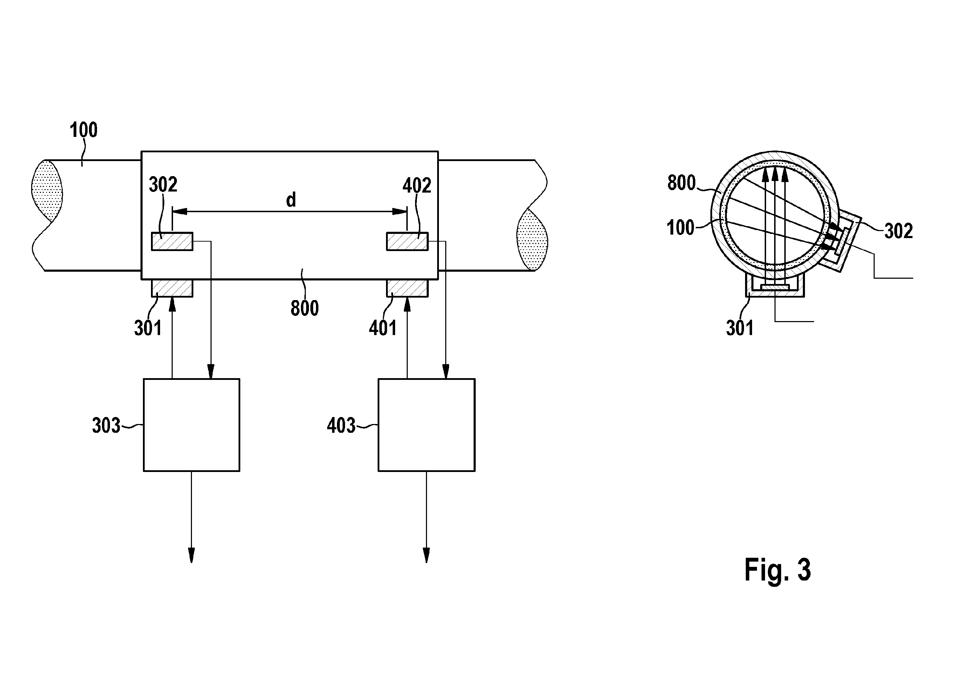

Fig. 3 zeigt eine alternative Anordnung von Sender 301 bzw. 401 und Empfangseinheit 302 bzw. 402 der jeweiligen Sensoranordnung an einer elastischen Manschette 800. Bei der in Fig. 3 abgebildeten Anordnung ist die jeweilige Empfangseinheit 302 bzw. 402 nicht so angeordnet, dass sie dem jeweiligen Sender 301 bzw. 401 direkt gegenüber liegt, sondern vielmehr so, dass die jeweilige Empfangseinheit 302 bzw. 402 quer zu einer Durchstrahlungsrichtung, beispielsweise infolge von Streuung an Blutteilchen auftretendes Licht erfasst. Dies geht besonders gut aus dem Querschnitt durch ein Blutgefäß 100 rechts in Fig. 3 hervor.

Die in Fig. 2 abgebildete Sensoranordnung erfasst somit eine Dämpfung des von einem jeweiligen Sender ausgesandten Lichtes durch die Blutteilchen, während die in Fig. 3 abgebildeten Sensoranordnungen durch die Blutteilchen gestreutes bzw. reflektiertes Licht erfassen.

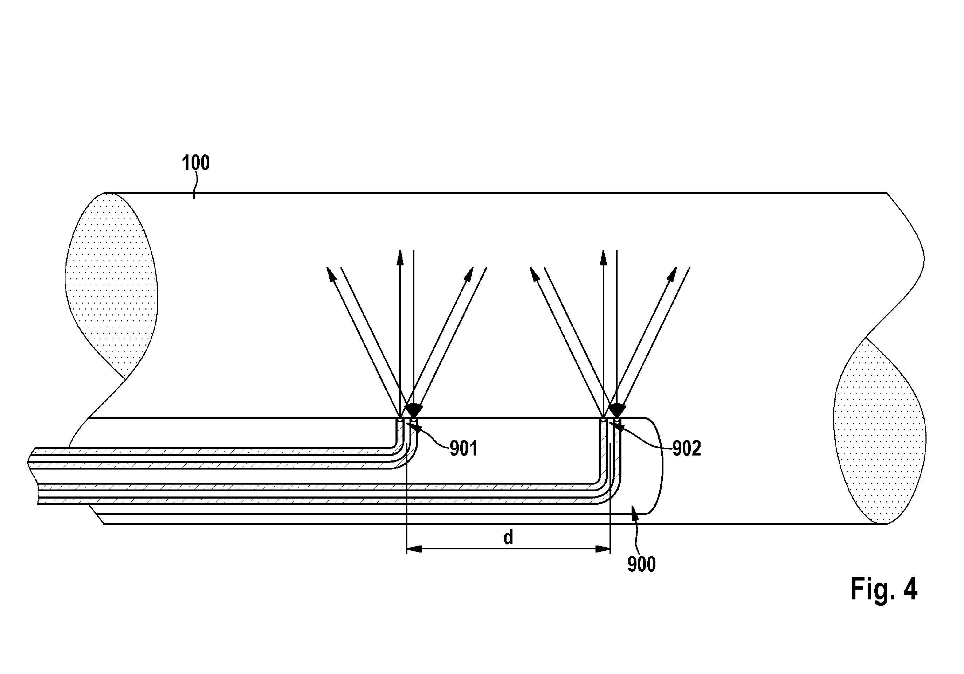

Fig. 4 zeigt eine andere Anordnung von Träger und Sensoranordnungen, nämlich in Form eines Katheters 900, der zum Einführen in ein Blutgefäß 100 ausgebildet ist. Das Ein- bzw. Auskoppeln von Licht in das Blut bzw. aus dem Blut geschieht dabei mit Hilfe von Lichtleitfasern 901 bzw. 902, die mit einem jeweiligen Sender bzw. einer jeweiligen Empfangseinheit eines entsprechenden Blutfluss-Sensors optisch verbunden sind.

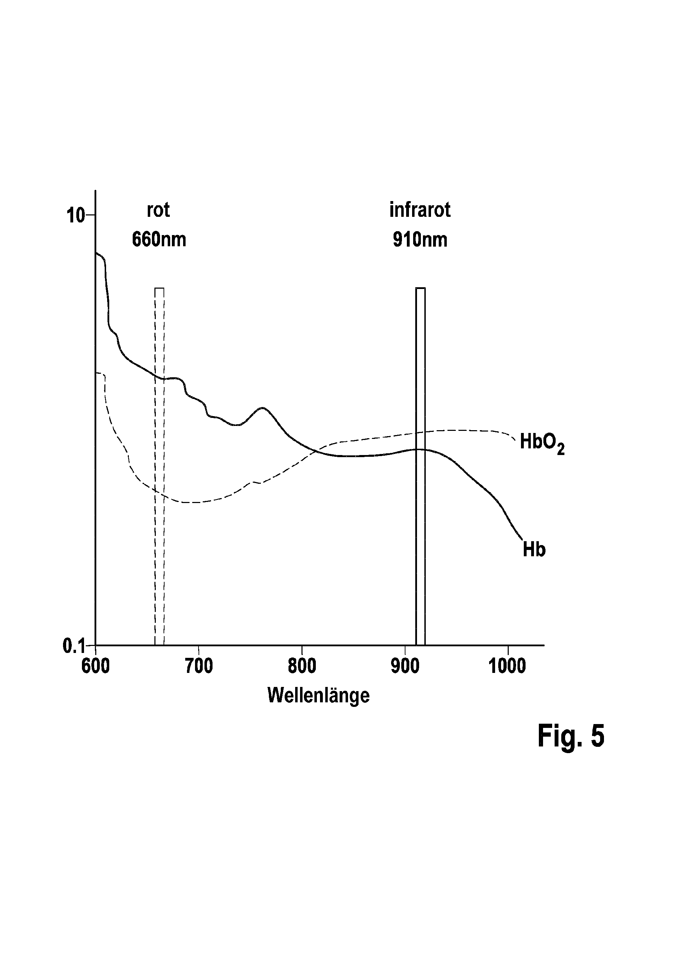

Besonders vorteilhaft ist es, wenn der Blutsensor gleichzeitig als Sensor für die Photoplethysmografie ausgebildet ist, so wie dies eingangs auch schon beschrieben ist. In diesem Falle besitzt wenigstens eine der Sensoranordnungen wenigstens zwei Sender, von denen einer Licht im roten Wellenlängenbereich, beispielsweise bei 660 nm, aussendet und der andere Sender Licht im infraroten Wellenbereich, beispielsweise bei 910 nm, aussendet. Fig. 5 zeigt die unterschiedlich starke Dämpfung von Licht bei verschiedenen Wellenlängen in Abhängigkeit der Blutsauerstoffverdickung.

Die erfindungsgemäß mögliche Messung und Auswertung der Fließgeschwindigkeit des Blutes alleine oder vorzugsweise in Kombination mit anderen Werten, wie dem Blutdruck, der Blutsauerstoffsättigung, Stimulationszeitpunkten, der Positur (Stehen, Sitzen oder Liegen) eines Patienten, eines Aktivitätssignals oder dergleichen erlaubt es gleichermaßen, ein differenzierteres Patienten-Monitoring zu betreiben oder eine verbesserte Herzschrittmachertherapie anzubieten. Im letztgenannten Sinne ist es besonders bevorzugt, wenn ein Blutfluss-Sensor der hier beschriebenen Art Bestandteil eines Herzstimulators ist oder mit diesem verbunden ist.

Es ergibt sich beispielsweise folgendes System (siehe auch Figur 1 und zugehörige Beschreibung): Ein implantierbares medizinisches Gerät (IMD; implantable medical device), zum Beispiel ein Schrittmacher, implantierbarer Kardioverter/Defibrillator (ICD) oder ein Monitoring-Implantat ist über Kabel mit einer elastischen Gefäßmanschette verbunden. Das IMD enthält eine Energiequelle (zum Beispiel eine Batterie), Einrichtungen zur Speisung der Lichtquellen (zum Beispiel Leuchtdioden, nachfolgend auch als LEDs bezeichnet), Einrichtungen zum Empfang und zur Filterung der Empfangssignale der optischen Empfangseinheit (zum Beispiel Fotodioden), Einrichtungen zur Steuerung des Messablaufs und zur Verarbeitung der Signale, zur Speicherung der Daten und für die Telemetrie mit einem externen Patientengerät. Weiterhin enthält das IMD eine Einrichtung zur Bestimmung der Kreuzkorrelationsfunktion aus zwei Empfangssignalen nach allgemein bekannten Methoden.

Die elastische Gefäßmanschette ist mit zwei Sensoranordnungen ausgerüstet, die in einem definierten Abstand (zum Beispiel 1 cm) angebracht sind. Jede dieser Sensoranordnungen enthält eine Lichtquelle aus zwei Leuchtdioden (LEDs; zum Beispiel für rotes und infrarotes Licht) und einer optischen Empfangseinheit mit zum Beispiel einer Fotodiode, die der Lichtquelle gegenüber angebracht ist. Die beiden LEDs in der Lichtquelle werden in kurzen Abständen alternierend für eine kurze Zeit (zum Beispiel 10 ms) eingeschaltet und mittels der Fotodiode wird das gegenüber einfallende Licht gemessen. Daraus wird für beide Wellenlängen jeweils die Lichtdämpfung beim Durchgang durch die Blutströmung und dann das Verhältnis der jeweiligen Lichtdämpfungen bei beiden Wellenlängen bestimmt. Das dadurch erhaltene Signal wird mit einem Bandpass gefiltert (zum Beispiel von 200 Hz bis 5 kHz), womit der Rauschanteil des Signals herausgelöst wird. Zeitgleich erfolgt eine identische Messung und Signalverarbeitung mit der zweiten Sensoranordnung.

Die beiden Rauschsignale der beiden Sensoranordnungen werden auf die Einrichtung zur Bestimmung der Kreuzkorrelationsfunktion (hier auch als Kreuzkorrelationseinheit 500 bezeichnet) geführt. Anhand der Maxima der Kreuzkorrelationsfunktion wird durch die Signalverarbeitungseinheit (zum Beispiel einen Microcontroller) den Zeitversatz der Rauschsignale ermittelt und daraus mittels des bekannten Abstands der beiden Sensoranordnungen die Blutflussgeschwindigkeit ermittelt. Die Blutflussgeschwindigkeiten werden über eine bestimmte Zeit, zum Beispiel einen Herzzyklus, im Speicher 603 der Signalverarbeitungseinheit (Steuer- und Auswerteeinheit 600) als Blutflusskurve abgelegt. Aus dieser Blutflusskurve können weitere diagnostische Parameter, wie zum Beispiel der maximale systolische Blutfluss oder der Anstieg der Blutflussgeschwindigkeit in der Systole bestimmt werden. Zur Unterdrückung von Störungen auf der Blutflusskurve kann diese auch synchron über mehrere Herzzyklen gemittelt werden. Die Blutflusskurve und/oder die daraus bestimmten Parameter können in bestimmten Intervallen über eine drahtlose Telemetrie vom IMD an ein Patientengerät und von diesem weiter an ein Telemonitoring-Servicecenter übertragen werden.

## Patentansprüche

1. Blutflusssensor für die Messung einer Blutflussgeschwindigkeit in einem Blutgefäß eines lebenden Körpers, mit
- einem Träger und wenigstens einem Sender zum Aussenden von Wellen, deren Ausbreitung von zellulären Bestandteilen des Blutes gegenüber der Ausbreitung im Blutplasma abgelenkt werden, und
- zwei an dem Träger mit einem Abstand voneinander befestigten Empfangseinheiten zum Empfangen von dem Sender ausgesandter Wellen,
wobei die Empfangseinheiten so angeordnet sind, dass die jeweils empfangenen Wellen im Einsatzfall einen voneinander verschiedenen, wenigstes in Strömungsrichtung des Blutes zueinander versetzten Weg durch das Blut genommen haben, sowie mit
- einer mit den Empfangseinheiten verbundenen Filtereinheit zum Filtern eines Ausgangssignals einer jeweiligen Empfangseinheit derart, dass das ein jeweiliges gefiltertes Ausgangssignal der Filtereinheit einen Rauschanteil eines jeweiligen Ausgangssignals einer jeweiligen Empfangseinheit repräsentiert,
- einer mit der Filtereinheit verbundenen Kreuzkorrelationseinheit zum Bestimmten eines Zeitversatzes zwischen dem gefilterten Ausgangssignals einer Empfangseinheit und dem gefilterten Ausgangssignal der jeweils anderen Empfangseinheit durch Korrelieren des gefilterten Ausgangssignals einer Empfangseinheit mit dem gefilterten Ausgangssignal der jeweils anderen Empfangseinheit, wobei der Zeitversatz umgekehrt proportional zur jeweiligen Blutflussgeschwindigkeit ist.

2. Blutflusssensor nach Anspruch 1, **dadurch gekennzeichnet, dass** der Sender zum Aussenden von Lichtwellen vorzugsweise im roten oder infraroten Wellenlängenbereich ausgebildet ist und dass die Empfangseinheiten zum Aufnehmen eines optischen Signals vorzugsweise im roten oder infraroten Wellenlängenbereich und zum Wandeln des aufgenommenen optischen Signal in ein elektrisches Signal ausgebildet sind.

3. Blutflusssensor nach Anspruch 1 oder 2, **dadurch gekennzeichnet, dass** der Sender eine Leuchtdiode als Lichtquelle enthält.

4. Blutflusssensor nach einem der Ansprüche 1 bis 3, **dadurch gekennzeichnet, dass** der Sender eine Lichtquelle und eine mit dieser optisch gekoppelte Lichtleitfaser enthält, welche angeordnet und ausgebildet ist, von der Lichtquelle stammendes Licht im Einsatzfall in ein Blutgefäß zu richten.

5. Blutflusssensor nach einem der Ansprüche 1 bis 4, **dadurch gekennzeichnet, dass** die Empfangseinheiten jeweils eine Photodiode enthalten.

6. Blutflusssensor nach einem der Ansprüche 1 bis 5, **dadurch gekennzeichnet, dass** der Blutflusssensor zwei Sender aufweist, von denen jeweils einer je einer Empfangseinheit zugeordnet und so angeordnet ist, dass vom jeweiligen Sender abgegebene Wellen im Einsatzfall nach Streuung, Reflexion oder Transmission durch einen Blutstrom auf die jeweils zugeordnete Empfangseinheit treffen.

7. Blutflusssensor nach einem der Ansprüche 1 bis 6, **dadurch gekennzeichnet, dass** der Blutflusssensor zwei Sender aufweist, die einer Empfangseinheit oder zwei Empfangseinheiten zugeordnet und so angeordnet sind, dass vom jeweiligen Sender abgegebene Wellen im Einsatzfall nach Streuung, Reflexion oder Transmission durch einen Blutstrom auf die jeweils zugeordnete Empfangseinheit treffen, wobei die Sender ausgebildet sind, Licht voneinander verschiedener Wellenlänge auszusenden und wobei die Empfangseinheit oder die Empfangseinheiten mit einer Auswerteeinheit verbunden ist, die zur Auswertung eines Ausgangssignals der Empfangseinheit im Sinne der Photoplethysmografie durch Bestimmung eines Verhältnisses zweier Dämpfungen für von den Sendern ausgesandtes Licht unterschiedlicher Wellenlänge ausgebildet ist.

8. Blutflusssensor nach einem der Ansprüche 1 bis 7, **dadurch gekennzeichnet, dass** der Träger eine elastische Manschette ist, die ausgebildet ist, um ein Blutgefäß gelegt zu werden.

9. Blutflusssensor nach Anspruch 8, **dadurch gekennzeichnet, dass** die elastische Manschette mit Dehnungsmessmitteln versehen ist, die ausgebildet sind ein eine elastische Dehnung der Manschette repräsentierendes Dehnungssignal zu liefern.

10. Blutflusssensor nach einem der Ansprüche 1 bis 7, **dadurch gekennzeichnet, dass** der Träger ein Katheter ist, der ausgebildet ist, in ein Blutgefäß eingeführt zu werden.

11. Blutflusssensor nach einem der Ansprüche 1 bis 10, **dadurch gekennzeichnet, dass** die Filtereinheit einen Bandpassfilter enthält, dessen Passband dem Frequenzbereich eines durch Blutteilchen verursachten Rauschanteils des Ausgangssignals einer jeweiligen Empfangseinheit entspricht.

12. Blutflusssensor nach einem der Ansprüche 1 bis 11, **dadurch gekennzeichnet, dass** der Blutflusssensor mit einer Steuer- und Auswerteeinheit verbunden ist, die ausgebildet ist, erfasste Strömungsgeschwindigkeiten über mehrere Herzzyklen zu mitteln.

13. Implantierbares medizinisches Gerät mit einem Blutflusssensor gemäß einem der Ansprüche 1 bis 12.

14. Gerät nach Anspruch 13, **dadurch gekennzeichnet, dass** das Gerät ein Monitoring-Implantat oder ein Herzstimulator mit einer Telemetrieeinheit zur Datenübertragung ist, welche mit dem Blutflusssensor wenigstens indirekt verbunden ist.
